# EUROPEAN PATENT APPLICATION

(11) **EP 3 097 852 A1**
(43) Date of publication of application: **30.11.2016**
(21) Application number: 15740717.2
(22) Date of filing: 26.01.2015
(51) Int. Cl.: A61B 6/00, G03B 42/04

(54) **RADIATION IMAGE DETECTOR, AND HOUSING FOR RADIATION IMAGE DETECTOR**

(30) Priority: 24.01.2014 KR 20140008977
(71) Applicant: Rayence Co., Ltd., Hwaseong-si, Gyeonggi-do 445-170 (KR); Vatech Ewoo Holdings Co., Ltd, Gyeonggi-do 445-170 (KR)
(72) Inventor: KIM, Nam Hyeong, Hwaseong-si Gyeonggi-do 445-170 (KR); KIM, Jae Hong, Seoul 158-090 (KR); PARK, Se Hee, Hwaseong-si Gyeonggi-do 445-170 (KR)
(74) Representative: Schmid, Wolfgang
(86) International application number: PCT/KR2015/000814
(87) International publication number: WO 2015/111983

(57) **Abstract**

The present invention relates to a radiography detector and a housing for the same. The radiography detector may include: a plate-shaped detector assembly for detecting radiation; a housing body formed of a carbon fiber and having a seamless structure in which a detector entrance/exit port through which the detector assembly is inserted and taken out is formed only at one side thereof; and a housing cover detachably coupled to one side of the housing body so as to open/close the detector entrance/exit port.The radiography detector and the housing for the same may have a simple and slim structure and a small weight. Furthermore, the radiography detector and the housing for the same may provide convenience of use and have a satisfactory appearance.

## Description

### Technical Field

The present disclosure relates to a radiography detector and a housing for the same, and more particularly, a radiography detector which has a simple and slim structure and a small weight, and a housing for the same.

### Background Art

A conventional radiograph detection method which is widely used for medical use, such as an X-ray examination method, must perform radiography using a detection film, and develop the film to determine the result.

With the recent development of semiconductor technology, an FPD (Flat Panel Detector) for obtaining digital image data from irradiated radiation has been suggested as a detector for obtaining a radiograph for medical use.

Japanese Patent Publication No. 2002-311527 has disclosed a radiography detector which has portability, mobility and ease of use.

The conventional radiography detector has a structure in which a detector assembly for detecting a radiograph is housed in a housing, and the detector assembly includes a detector panel and a driving circuit board for detecting radiation to obtain an image signal and a power supply unit for supplying a driving voltage.

In the conventional radiography detector, radiation must be transmitted to the detector assembly through the housing, and stiffness for external shock or unexpected fall must be considered. Thus, the housing is formed of a magnesium material which exhibits satisfactory strength while having radiation transparency.

The housing includes a top housing and a bottom housing which are coupled to each other and separated from each other, such that the detector assembly can be housed in the housing or taken out of the housing. Furthermore, the housing includes a depressed or opened battery coupling part formed in a part of the bottom housing. The battery coupling part serves as a power supply unit, and includes a power connector.

However, the conventional radiography detector has a limitation in reducing weight because the housing is formed of magnesium. On the other hand, when the thickness of the housing is decreased in order to reduce weight, the magnesium material cannot guarantee stiffness, and the housing may be deformed.

Furthermore, since the top housing and the bottom housing are coupled to each other in the vertical direction, the thickness of side portions thereof is inevitably increased in order to secure the stiffness of the coupling portion therebetween. Therefore, the conventional radiography detector has a limitation in reducing weight.

Moreover, since a battery is coupled to the battery coupling part formed in the bottom housing, the battery may be exposed to the outside while forming a part of the appearance. Thus, the radiography detector does not have a simple structure. On the other hand, when the battery is separated, the depressed structure of the battery coupling part may be exposed to degrade the appearance.

US Patent Application 8,035,083 has disclosed a cassette type radiographic image solid-state detector which includes a housing constituted of a carbon fiber, in order to reduce weight and to secure structural stiffness. This radiographic image solid-state detector has a structure in which a detector assembly is housed in a tube-shaped housing body of which both ends are opened, and both ends of the housing body are opened and closed through first and second cover members.

The radiographic image solid-state detector according to US Patent Application 8,035,083 can reduce weight and secure structural stiffness because the housing is constituted of a carbon fiber. However, since the radiographic image solid-state detector includes two covers for opening/closing both ends of the housing body, the radiographic image solid-state detector does not have a simple structure, and has a limitation in reducing weight due to the number of covers, while the appearance thereof is degraded.

### Detailed Description of the Invention

### Technical Problem

Various embodiments are directed to a radiography detector which has a simple and slim structure and a small weight, and a housing for the same.

Also, various embodiments are directed to a radiography detector which provides convenience of use and has a satisfactory appearance, and a housing for the same.

### Technical Solution

In an embodiment, a radiography detector may include: a plate-shaped detector assembly for detecting radiation; a housing body having a detector entrance for entrance and exit of the detector assembly at one side thereof and formed in a seamless shape using a carbon fiber; and a housing cover detachably coupled to one side of the housing body to open/close the detector entrance.

The detector assembly may have a battery slot formed at one region thereof, the battery slot being opened toward the outside, and the housing body or the housing cover may have a battery entrance formed at one side thereof, the one side corresponding to the opening of the battery slot.

An insertion guide groove and an insertion guide protrusion corresponding to each other in shapes may be formed at both sides of the battery in the widthwise direction and both sides of the battery entrance and the battery slot in the widthwise direction, respectively, along the insertion direction.

The opening of the battery slot may be formed in a direction facing the housing cover, and the battery entrance may be formed in the housing cover.

The radiography detector may further include a battery cover for opening and closing the battery entrance.

The radiography detector may further include a battery discharge unit installed in the battery slot, and elastically pressurizing the battery inserted into the battery slot to the outside when the battery cover is opened.

The radiography detector may further include a battery discharge unit installed in the battery slot, and elastically pressurizing the battery inserted into the battery slot to the outside when the battery cover is opened.

The buffer member may include one or more of an end buffer member formed on all or part of side surfaces of the detector assembly and a surface buffer member formed on all or part of the top and bottom surfaces of the detector assembly.

The surface buffer member may include a protective sheet installed on the outer surface thereof and coming in sliding contact with the inner surface of the housing body.

In another embodiment, there is provided a housing for a radiography detector, which houses a plate-shaped detector assembly of the radiography detector. The housing may include: a housing body having a detector entrance for entrance and exit of the detector assembly at one side thereof and formed in a seamless shape using a carbon fiber; and a housing cover detachably coupled to one side of the housing body so as to open/close the detector entrance.

### Advantageous Effects

According to the embodiments of the present invention, the radiography detector and the housing for the same may have a simple and slim structure and a small weight.

Furthermore, the radiography detector and the housing for the same may provide convenience of use and have a satisfactory appearance.

### Brief Description of Drawings

FIG. 1 is a perspective view of a radiography detector according to a first embodiment of the present invention.
FIG. 2 is an exploded perspective view of the radiography detector of FIG. 1.
FIG. 3 is a perspective view of a detector assembly of the radiography detector of FIG. 1.
FIG. 4 is a perspective view of a radiography detector according to a second embodiment of the present invention.
FIG. 5 is an exploded perspective view of the radiography detector of FIG. 4.
FIG. 6 is a perspective view of a detector assembly of the radiography detector of FIG. 5.
FIG. 7 is an expanded perspective view of a battery slot of FIG. 6.
FIGS. 8 and 9 are expanded views of a battery entrance of FIGS. 4 and 5.

### Best Mode for Carrying Out the Invention

Hereafter, exemplary embodiments of the present invention will be described in detail with reference to the accompanying drawings.

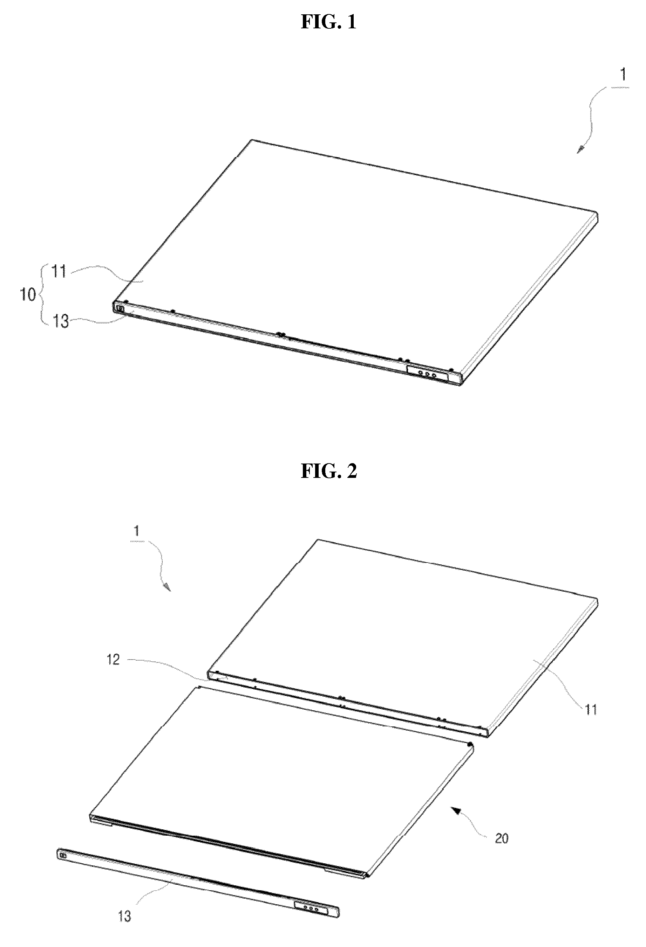

FIGS. 1 to 3 are perspective views of a radiography detector according to a first embodiment of the present invention. As illustrated in FIGS. 1 to 3, the radiography detector 1 according to the first embodiment of the present invention includes a housing 10 and a detector assembly 20. The housing 10 covers the detector assembly 20 and forms the appearance of the radiography detector, and the detector assembly 20 is inserted into the housing 10 or taken out from the housing 10.

The housing 10 includes a housing body 11 for housing the detector assembly 20 and a housing cover 13 for opening/closing the entrance area of the detector assembly 20 in the housing body 11.

The housing body 11 is formed of a carbon fiber which is light, exhibits excellent stiffness, and has radiation transparency, and has a rectangular slim plate-shaped hollow body. The housing body 11 has a detector entrance 12 for entrance/exit of the detector assembly 20, the detector entrance being formed at one side of the circumference of the housing body 11. The plate-shaped hollow body of the housing body 11 is implemented as a single body structure. That is, the housing body 11 has a seamless structure with no seams or connections between the respective surfaces. Therefore, the radiography detector 1 has a slim structure with a simple and smooth appearance.

The housing cover 13 has an area corresponding to the thickness of the housing body 11 and the detector entrance 12, and opens /closes the detector entrance 12. The housing cover 13 is detachably coupled to the housing body 11 through a plurality of small screws. Thus, although the direction of the radiography detector 1 is switched to an arbitrary direction, the detector assembly 20 housed in the housing body 11 can be prevented from coming off. The housing cover 13 may be not only coupled to the housing body 11 through the screws, but also coupled to the housing body 11 through various coupling structures such as a hinge structure and sliding structure, in order to open/close the detector entrance 12.

The housing cover 13 may include at least a part of various elements such as a power switch and an operation state display unit of the radiography detector 1 and an antenna of a communication unit. Furthermore, the housing cover 13 may include an external device connector or the like. As the above-described elements are included in the housing cover 13 having a slim area, the simpleness of the appearance of the radiography detector 1 may be improved. The elements may be installed in at least one region of the housing body 11 instead of the housing cover 13, as long as they do not hinder radiation transmission.

As described above, the single housing cover 13 having a slim area closes the detector entrance 12 so as to be integrated with the housing body 11. Thus, the housing cover 13 improves the simpleness of the appearance of the radiography detector 1.

The housing 10 of the radiography detector 1 can be manufactured as a single housing product capable of housing various types of detector assemblies 20, as long as they can be housed in the housing 10 and detect a radiograph.

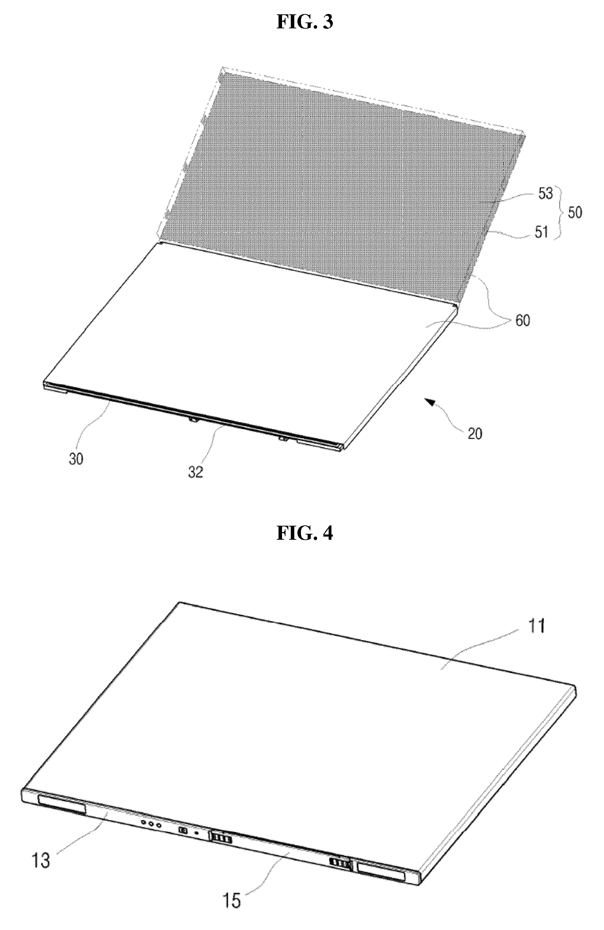

As illustrated in FIG. 3, the detector assembly 20 includes a detector unit 30, a detector buffer member 50 and a radiation-transparent protective sheet 60. The detector unit 30 receives radiation incident through the housing body 11 and generates a detection image. The detector buffer member 50 protects the detector unit 30 from shock transmitted from outside, and fills the gap between the detector unit 30 and the housing 10. The radiation-transparent protective sheet 60 protects the detector buffer member 50 and the detector unit 30 while minimizing a frictional force which is caused by the detector buffer member 50 while the detector unit 30 is inserted into or taken out of the housing body 11.

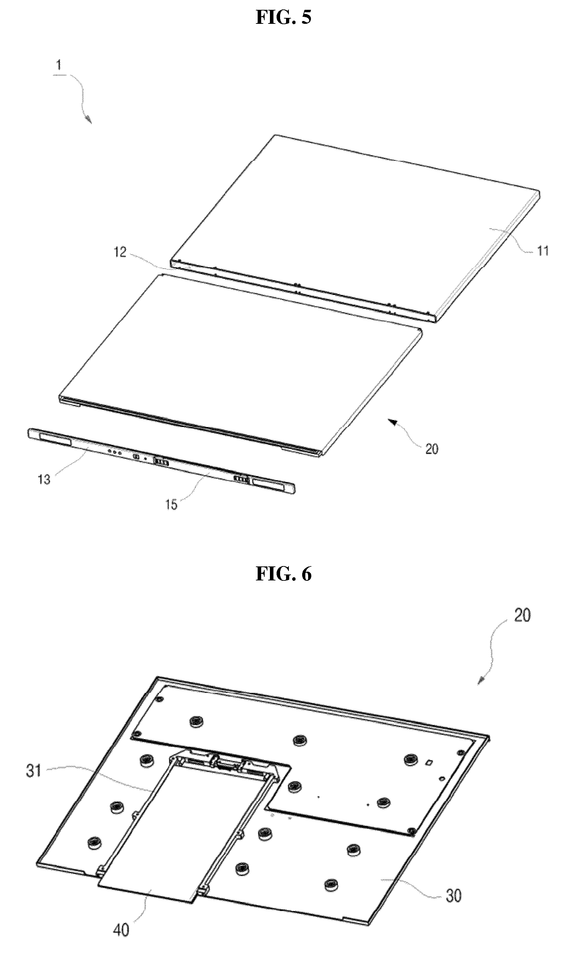

The detector unit 30 has an area corresponding to the housing body 11, and includes a detector panel, a driving circuit board, a battery (refer to reference number 40 of FIG. 6) and a communication unit. At this time, the detector unit 30 may have a battery slot 32 in which the battery 40 is detachably coupled, in consideration of the replacement of the battery 40. The battery 40 may be formed in a plate shape corresponding to the slim structure of the radiography detector 1.

The detector buffer member 50 has an end buffer member 51 and a surface buffer member 53. The end buffer member 51 is installed at both ends of the detector assembly 20, corresponding to both sides of the detector entrance 12 in the longitudinal direction, and the surface buffer member 53 is installed on the top and bottom surfaces of the detector assembly 20.

The end buffer member 51 and the surface buffer member 53 may be formed of various materials, as long as they can smoothly transmit radiation while being elastically compressed and expanded. Desirably, the end buffer member 51 and the surface buffer member 53 may be formed of EVA (Ethylene-Vinyl Acetate).

At this time, the end buffer member 51 may be installed so as to correspond to the entire circumference of the detector unit 30, or a plurality of end buffer members 51 may be installed at positions which are separated from each other along the circumferential direction of the detector unit 30. The end buffer member 51 may be attached to the inner circumferential surface of the protective sheet 60. The surface buffer member 53 has an area capable of covering the top and bottom surfaces of the detector unit 30. The surface buffer member 53 may be attached or not attached to the top and bottom inner surfaces of the protective sheet 60.

The protective sheet 60 may be folded or unfolded to cover the top and bottom surfaces and the circumference of the detector unit 30, and formed of a radiation-transparent film sheet. Desirably, the protective sheet 60 may be formed of a polycarbonate (PC) film having a coefficient of kinetic friction at which the protective sheet 60 can slide on the inner surface of the housing body 11 with a material characteristic of easy radiation transmission. In addition to the PC film, the protective sheet 60 may include various types of film sheets as long as they have the above-described coefficient of kinetic friction with a material characteristic of easy radiation transmission.

The radiography detector 1 and the housing 10 for the same according to the first embodiment of the present invention have a structure in which the detector assembly 20 is housed in the housing 10 that is formed of carbon fiber and has a plate-shaped hollow structure with only the detector entrance 12 at one side thereof. Thus, the radiography detector 1 and the housing 10 for the same may have a simple and slim appearance while the weight thereof is reduced.

Furthermore, as the detector assembly 20 is inserted into or taken out from the housing 10 through the detector entrance 12 which is opened and closed by the housing cover 13, the radiography detector 1 and the housing 10 for the same can provide convenience of use and exclude unnecessary external parts. Thus, the radiography detector 1 and the housing 10 for the same may have a satisfactory appearance.

FIGS. 4 to 9 are perspective views of a radiography detector according to a second embodiment of the present invention. As illustrated in FIGS. 4 to 9, the radiography detector according to the second embodiment of the present invention includes a housing 10 and a detector assembly 20 as almost the same components as the first embodiment of the present invention. The radiography detector according to the second embodiment of the present invention may further include a structure and components for entrance of the battery 40. Therefore, the duplicated descriptions of the housing 10 and the detector assembly 20 are omitted herein, and the following descriptions will be focused on the structure and components for entrance of the battery 40.

In the radiography detector 1 according to the second embodiment of the present invention, the structure for entrance/exit of the battery 40 is formed at the detector entrance 12 of the housing 10. For this structure, the housing cover 13 has a battery entrance 14 formed therein, and the detector unit 30 of the detector assembly 20 has a battery slot (refer to 32 of FIG. 3) opened to the battery entrance 14 of the housing cover 13.

The housing cover 13 may include a battery cover 15 for opening/closing the battery entrance 14. The battery cover 15 prevents separation of the battery 40, and blocks the battery 40 from being exposed to the outside such that the appearance of the radiography detector 1 is not degraded.

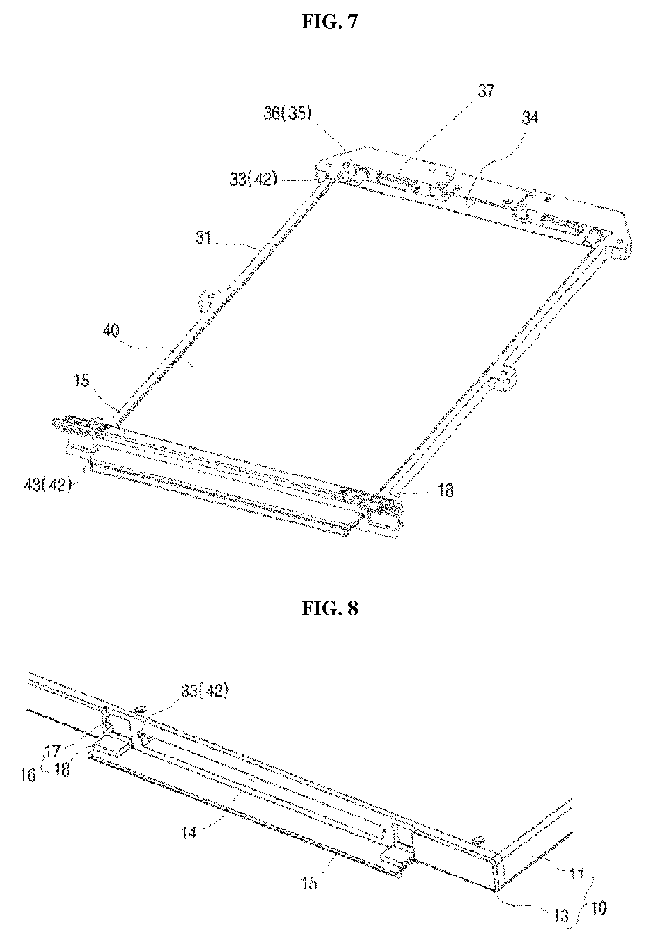

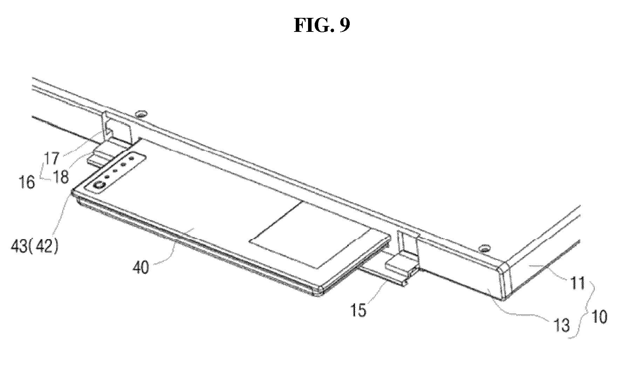

At this time, as illustrated in FIGS. 8 and 9, the battery cover 15 may be hinge-coupled to the battery entrance 14 of the housing cover 13, and turned to open/close the battery entrance 14. The opening/closing structure of the battery cover 15 may include various types of opening/closing structures such as a detachable opening/closing structure and a sliding opening/closing structure.

The battery cover 15 may further include a locking unit 16 which prevents the battery entrance 14 from being arbitrarily opened by the movement or direction change of the radiography detector 1 or external shock. The locking unit 16 of the battery cover 15 may include various structures as long as they can be locked or unlocked at the circumferential area of the battery entrance 14 and the circumferential area of the battery cover 15. As illustrated in FIGS. 8 and 9, the locking unit 16 may include a locking groove 17 and a locking piece 18 which are formed in the circumferential area of the battery entrance 14 and the circumferential area of the battery cover 15, respectively. The locking piece 18 is locked to or unlocked from the locking groove 17. In addition, the locking unit 16 may include a hook coupling structure which is locked or unlocked while the battery cover 15 is opened/closed. When the battery cover 15 is detachably coupled to the housing cover 13, the locking unit 16 may include a plurality of small screws and screw holes.

As illustrated in FIGS. 6 and 7, the battery slot 32 of the detector assembly 20 may be formed by coupling a separate guide block 31 to the detector unit 30. If necessary, the detector unit 30 may have a battery housing space serving as the battery slot 32.

The battery slot 32 has a relatively large area while being thinner than the detector unit 30, and the battery 40 is provided as a thin-plate large-capacity battery corresponding to a thickness and an area of the battery slot 32.

The battery slot 32 has a power connector 34 connected to a power terminal (not illustrated) of the battery 40. At this time, the power terminal of the battery 40 and the power connector 34 of the battery slot 32 may be formed at the insertion-side rear end of the battery 40 and the inner rear of the battery slot 32, respectively. Thus, the power terminal of the battery 40 and the power connector 34 of the battery slot 32 may be naturally connected to each other while the battery 40 is inserted.

The battery slot 32 may include a battery discharge unit 35 formed at the inner rear thereof, and the battery discharge unit 35 elastically pressurizes the battery 40 inserted into the battery slot 32 to the outside, when the battery cover 15 is opened.

The battery discharge unit 35 may include a pressurizing pin 36 and a spring (not illustrated). The pressurizing pin 36 protrudes from the guide block 31 at the inner rear of the battery slot 32 and comes in contact with the insertion-side rear end of the battery 40, and the spring elastically pressurizes the pressurizing pin 36 in the direction where the pressurizing pin 36 protrudes. The battery discharge unit 35 elastically pressurizes the battery 40 inserted into the battery slot 32 to the outside of the battery entrance 14 such that the battery 40 protrudes to the outside of the housing cover 13. Thus, a user can easily take out the battery 40.

The spring of the battery discharge unit 35 is compressed in a state where the battery 40 is inserted into the battery slot 32. Then, when the user presses the battery 40, the spring is expanded to push the pressurizing pin 36. Thus, the battery 40 protrudes to the outside of the battery entrance 14.

Alternatively, when the battery entrance 14 is closed by the battery cover 15 after the battery 40 is inserted into the battery slot 32, the spring is compressed. Then, when the battery cover 15 is opened, the spring is expanded to push the pressurizing pin 36. Thus, the battery 40 protrudes to the outside of the battery entrance 14.

The battery slot 32 may further include a battery buffer member 37 for buffering insertion shock which is applied to the battery 40 when the battery 40 is inserted into the battery slot 32. The battery buffer member 37 is formed at the inner rear of the battery slot 32, and buffers contact shock between the insertion-side rear end of the battery 40 and the inner rear wall of the battery slot 32 when the battery 40 is inserted, thereby preventing damage of the battery 40. The battery buffer member 37 may be formed of various materials such as rubber, silicon and synthetic resin sheet having abrasion resistance.

The battery slot 32, the battery entrance 14, and the battery 40 may include a battery insertion guide structure 42 for preventing mis-insertion of the battery 40.

The battery insertion guide structure 42 includes an insertion guide groove 33 and an insertion guide protrusion 43 which are formed along the insertion direction. The insertion guide groove 33 is formed at one between both sides of the battery 40 in the widthwise direction and both sides of the battery entrance 14 and the battery slot 32 in the widthwise direction. The insertion guide protrusion 43 is formed at the other between both sides of the battery 40 in the widthwise direction and both sides of the battery entrance 14 and the battery slot 32 in the widthwise direction, and has a shape corresponding to the insertion guide groove 33. At this time, the insertion guide protrusion 43 and the insertion guide groove 33 may have a stepped cross-section structure or uneven cross-section structure, when seen from the battery entrance 21.

Although not illustrated, the structure and components for entrance and exit of the battery 40, such as the battery slot 32 and the battery cover 15, may be installed at one region in the opposite side of the circumference of the housing body 11, instead of the housing cover 13. At this time, the battery slot 32, the battery cover 15 and the battery 40 may have the same structures as described above, except the positions thereof.

The radiography detector 1 and the housing 10 for the same according to the second embodiment of the present invention has a simple and slim appearance and a small weight, provides convenience of use, and excludes unnecessary external parts, thereby having a satisfactory appearance, like the first embodiment of the present invention.

Furthermore, the entrance structure and components of the battery 40 provide a structure capable of enabling a user to easily replace the battery 40 without degrading the appearance. Moreover, the entrance/exit structure and components of the battery 40 can prevent mis-insertion of the battery 40 and provide convenience in taking out the battery 40, while the battery 40 is provided as a thin-plate large-capacity battery.

According to the embodiments of the present invention, the radiography detector and the housing for the same may have a simple and slim structure and a small weight.

Furthermore, the radiography detector and the housing for the same may provide convenience of use and have a satisfactory appearance.

### Industrial Applicability

The present invention can be used for a radiography detector for medical use.

## Claims

1. A radiography detector comprising:
a plate-shaped detector assembly for detecting radiation;
a housing body having a detector entrance for entrance and exit of the detector assembly at one side thereof and formed in a seamless shape using a carbon fiber; and
a housing cover detachably coupled to one side of the housing body to open and close the detector entrance.

2. The radiography detector of claim 1, wherein the detector assembly has a battery slot formed at one region thereof, the battery slot being opened toward the outside, and
the housing body or the housing cover has a battery entrance formed at one side thereof, the one side corresponding to the opening of the battery slot.

3. The radiography detector of claim 2, wherein an insertion guide groove and an insertion guide protrusion corresponding each other in shapes at both sides of the battery in the widthwise direction and both sides of the battery entrance and the battery slot in the widthwise direction, respectively, along the insertion direction.

4. The radiography detector of claim 3, wherein the opening of the battery slot is formed in a direction facing the housing cover, and
the battery entrance is formed in the housing cover.

5. The radiography detector of any one of claims 2 to 4, further comprising a battery cover for opening/closing the battery entrance.

6. The radiography detector of claim 5, further comprising a battery discharge unit installed in the battery slot and elastically pressurizing the battery inserted into the battery slot to the outside when the battery cover is opened.

7. The radiography detector of claim 1, further comprising a buffer member interposed between the detector assembly and the inner surface of the housing body.

8. The radiography detector of claim 7, wherein the buffer member comprises one or more of an end buffer member formed on all or part of side surfaces of the detector assembly and a surface buffer member formed on all or part of the top and bottom surfaces of the detector assembly.

9. The radiography detector of claim 8, wherein the surface buffer member comprises a protective sheet installed on the outer surface thereof and coming in sliding contact with the inner surface of the housing body.

10. A radiography detector housing radiography detectorstoring a plate-shaped detector assembly of the radiography detector, comprising:
a housing body having a detector entrance for entrance and exit of the detector assembly at one side thereof and formed in a seamless shape using a carbon fiber; and
a housing cover detachably coupled to one side of the housing body so as to open and close the detector entrance.
